# EUROPEAN PATENT APPLICATION

(11) **EP 1 897 937 A1**
(43) Date of publication of application: **12.03.2008**
(21) Application number: 06018791.1
(22) Date of filing: 07.09.2006
(51) Int. Cl.: C12N 5/06, A61P 25/16

(54) **Method of culturing NPCs**

(71) Applicant: NeuroProgen GmbH Leipzig, 04103 Leipzig (DE)
(72) Inventor: Schwarz, Sigrid, 04316 Leipzig (DE)
(74) Representative: Kalhammer, Georg

(57) **Abstract**

The present invention provides an improved method for culturing neural precursor cells (NPCs). The method allows the proliferation and/or the dopaminergic differentiation of the NPCs by contacting them with at least one uracil nucleotide. The cells obtained by the method of the invention are useful in the treatment of neurodegenerative disorders.

SEQUENCE LISTING

<110>  NeuroProgen GmbH Leipzig

<120>  Method of culturing NPCs

<130>  NeuroProgen-uracil nucleotides

<160>  12

<170>  PatentIn version 3.3

<210>  1
<211>  17
<212>  DNA
<213>  Artificial

<220>
<223>  P2Y1 primer 1

<400>  1
gagggcccgg cttgatt                                                    17

<210>  2
<211>  22
<212>  DNA
<213>  Artificial

<220>
<223>  P2Y1 primer 2

<400>  2
atacgtggca taaaccctgt ca                                              22

<210>  3
<211>  21
<212>  DNA
<213>  Artificial

<220>
<223>  P2Y2 primer 1

<400>  3
tggtgcgctt cctcttctac a                                               21

<210>  4
<211>  17
<212>  DNA
<213>  Artificial

<220>
<223>  P2Y2 primer 2

<400>  4
accggtgcac gctgatg                                                    17

<210>  5
<211>  19
<212>  DNA
<213>  Artificial

<220>
<223>  P2Y2 primer 3

<400>  5

tcatggctcg tcgcctgta                                                    19

<210>  6
<211>  19
<212>  DNA
<213>  Artificial

<220>
<223>  P2Y2 primer 4

<400>  6
agagagcgga ggcgagaag                                                    19

<210>  7
<211>  18
<212>  DNA
<213>  Artificial

<220>
<223>  P2Y6 primer 1

<400>  7
cctgcccaca gccatctt                                                     18

<210>  8
<211>  21
<212>  DNA
<213>  Artificial

<220>
<223>  P2Y6 primer 2

<400>  8
cagtgagagc catgccatag g                                                 21

<210>  9
<211>  20
<212>  DNA
<213>  Artificial

<220>
<223>  P2X4 primer 1

<400>  9
ttgtgtggga aaagggctac                                                   20

<210>  10
<211>  20
<212>  DNA
<213>  Artificial

<220>
<223>  P2X4 primer 2

<400>  10
cctgtgtctg gttcatggtg                                                   20

<210>  11
<211>  20
<212>  DNA
<213>  Artificial

<220>

<223>  GAPDH primer 1

<400>  11
gctagtggtg gacctgacct                                                    20


<210>  12
<211>  20
<212>  DNA
<213>  Artificial

<220>
<223>  GAPDH primer 2

<400>  12
tgctgtagcc aaattcgttg                                                    20

## Description

The present invention provides an improved method for culturing neural precursor cells (NPCs). The method allows the proliferation and/or the dopaminergic differentiation of the NPCs. The cells obtained by the method of the invention are useful in the treatment of neurodegenerative disorders.

Disorders of the central nervous system (CNS) encompass numerous afflictions such as neurodegenerative diseases, acute brain injury and a large number of CNS dysfunctions. In recent years neurodegenerative diseases have become an important concern due to the expanding elderly population which is at the greatest risk for these disorders. The diseases which include for example Alzheimer's disease and Parkinson's disease are linked to the degeneration of neuronal cells in particular locations of the CNS leading to the inability of these cells, or the brain region, to carry out their intended function.

Unfortunately, the ability of the adult functional nervous system to produce new neural cells is quite limited as neurogenesis in mammals is largely completed early in the postnatal period. This relative inability to produce new neural cells in most mammals (and especially in primates) may be advantageous for long-term memory retention, however it is a distinct disadvantage when the need to replace lost neuronal cells arises due to an injury or disease.

To date treatment for CNS disorders is primarily carried out via the administration of pharmaceutical compounds. Unfortunately this type of treatment has shown limited efficacy and many complications including limited ability to transport drugs across the blood-brain-barrier and drug tolerance acquired by patients to whom these drugs are administered long-term. For instance partial restoration of dopaminergic activity in Parkinson's patients has been achieved with "levodopa" (L-DOPA) which is a dopamine precursor able to cross the blood-brain-barrier. However, patients become tolerant to the effects of "levodopa" and therefore steadily increasing dosages are needed to maintain its effects. In addition there are a number of side effects associated with "levodopa" such as increased and uncontrollable movement.

Recently it was found that the concept of neurological tissue transplantation can be applied to the treatment of neurological diseases such as Parkinson's disease. Neural grafts may avert the need not only for constant drug administration but also for complicated drug delivery systems which arise due to the blood-brain-barrier. Especially NPCs promise treatment for currently incurable brain diseases and injuries. Based on their unique capacity to migrate throughout the brain and to target invading tumor cells, transplantation of NPCs offers a new potential therapeutic approach as a cell based delivery system for gene therapy in brain tumors (Shah et al., Neural precursor cells and their role in neuro-oncology, Dev Neurosci 26: 118-130; 2004).

However, there are limitations to this technique as well. Cells used for transplantation which carry cell surface molecules of a differentiated cell from another host can induce an immune reaction in the host. In addition cells must be at a stage of development in that they are able to form normal neural connections as neighbouring cells. For these reasons initial studies on neurotransplantation centered on the use of fetal (embryonic) cells: Several studies have shown improvements in patients with Parkinson's disease after receiving grafts of fetal CNS tissue.

Though those studies are encouraging, there are further problems associated with neurological tissue transplantation as - for example - the use of large quantities of aborted fetal tissue for the treatment of disease raises ethical considerations and political obstacles.

In addition there are serious doubts as to whether sufficient supply of fetal tissue would be available for transplantation.

There are further considerations as well: It exists for example a potential of contamination during fetal tissue preparation. Moreover the tissue may already be infected with bacteria or virus and thus requires extensive diagnostic testing for each fetus used. However, even diagnostic testing might not uncover all infected tissue. For example the successful diagnoses of HIV-free tissue is not guaranteed because antibodies to the virus are generally not present until several weeks after infection.

There is also the added problem that fetal CNS tissue is composed of more than one cell type and thus is not a well defined source of tissue. Specific applications mostly require specifically differentiated cells; for example for the treatment of Parkinson's disease only dopaminergic cells are required. The ability to tightly control differentiation or form homogenous populations of partially differentiated or terminally differentiated cells by differentiation in vitro has been proved problematic. Uncontrolled differentiation induces mixtures of pluripotent stem cells and partially differentiated stem/precursor cells corresponding to various cell lineages. When these fetal-derived stem cell mixtures are transplantated into a recipient tissue, the contaminating pluripotent stem cells can proliferate and differentiate to form tumors while partially differentiated stem and precursor cells can further differentiate to form a mixture of inappropriate and undesired cell types. It is well known from studies in animal models that tumors originating from contaminating pluripotent cells can cause catastrophic tissue damage and death. In addition pluripotent cells contaminating a cell transplant can generate various inappropriate stem cells, precursor cells and differentiated cell types in the donor which can lead to the formation of inappropriate tissues within a cell transplant. These outcomes cannot be tolerated for clinical applications in humans.

Therefore, uncontrolled fetal-derived stem cell differentiation makes the clinical use of fetal-derived cells in human cell therapies impossible. While currently available transplantation approaches represent a significant improvement over other available treatments for neurological disorders, the transplantated cells - and therefore also the culture conditions - still need to be optimized before this transplantation method can be used clinically.

It has proven difficult to culture NPCs over longer periods of time and at the same time to retain the differentiation potential of the NPCs. The rate of proliferation of human NPCs is limited (Ostenfeld et al. (2000) Exp Neurol. 164(1):215-226). Thus, extended expansion and modification of such cells is limited. Using low oxygen conditions, the applicants have for the first time developed culture systems that allow for expansion of > 50 passages without affecting the potential of such precursor cells to differentiate into neurons or glia. Such long-term culture systems also allow for partial differentiation and modulation of such cells via long-term application of novel small molecules (see WO 00/78931 A2, filed by Horst Peschel). Therefore, the percentage of specific cells (e. g. dopaminergic neurons) can be markedly increased. However, there is still the need for further improved methods of culturing NPCs in order to obtain better proliferation of NPCs and/or better differentiation of NPCs into neuronal cells, in particular into dopaminergic neuronal cells.

It is therefore an object of the present invention to overcome one or more of the difficulties or deficiencies associated with the prior art.

The inventors of the present application surprisingly found that uracil nucleotides are capable of stimulating human neural precursor cell proliferation and dopaminergic differentiation. It has further been found that dexamethasone is capable of stimulating human neural precursor cell proliferation and dopaminergic differentiation. The present invention therefore relates to a method of culturing neural precursor cells (NPCs), comprising culturing the NPCs in a medium containing at least one uracil nucleotide or dexamethasone. The invention further relates to a method for differentiating NPCs, comprising culturing the NPCs in a medium containing at least one uracil nucleotide or dexamethasone. Another aspect of the invention is a method for proliferating NPCs, comprising culturing the NPCs in a medium containing at least one uracil nucleotide. Another aspect of the invention is a method for propagating NPCs, comprising culturing the NPCs in a medium containing at least one uracil nucleotide. Another aspect of the invention is a method for growing NPCs, comprising culturing the NPCs in a medium containing at least one uracil nucleotide. Another aspect of this invention is a method of culturing NPCs, comprising contacting said NPCs with at least one uracil nucleotide. Yet another aspect of this invention is a method for increasing the number of NPCs, comprising adding at least one uracil nucleotide to NPCs in an amount effective to increase the number of the NPCs.

All embodiments described herein with respect to uracil nucleotide(s) can be applied to dexamethasone *mutatis mutandis.* That means dexamethasone can be used instead of or in addition to the uracil nucleotide(s) unless indicated otherwise.

A "Neural precursor cell" or "NPC", as used herein, is a cell that is capable of self-renewing and of differentiating into glial cells and neurons. Usually, non-differentiated NPCs express the marker molecules nestin, CD15, CD56, CD90, CD164, and NGFR, whereas they do not express CD45, CD105 (endoglin), CD109, and CD140b (PDGF-RB) [Vogel, W. et al. Heterogeneity among human bone marrow-derived mesenchymal stem cells and neural progenitor cells. J. Haematol. 88, 126-133 (2003); the disclosure of which is incorporated herein by reference]. The term "self-renewing" refers to the capability of a cell to undergo mitosis and to divide to produce two daughter cells.

Human midbrain derived NPCs further express the purinergic receptors P2Y₁, P2Y₄, P2Y₆, and P2X₄. (see table 1 infra). The NPCs do usually not express the molecules which are characterized as being "absent" in all of the four samples (hNPC-1 through -4) tested in table 1 infra.

Isolation and culturing of NPCs from rodent brain has been reported in the state of the art (Daadi und Weiss, Generation of tyrosine hydroxylase producing neurons from precurors of the embryonic and adult forebrain, J Neurosci, 19:4484-4497, 1999; Liepelt et al., Differentiation potential of a monoclonal antibody-defined neural progenitor cell population isolated from prenatal rat brain by fluorescence-activated cell sorting, Brain Res Dev Brain Res, 51:267-278, 1999).

NPCs can be isolated from fetal (embryonic) or adult brain or spinal cord preparations. If an adult donor is used NPCs are preferably isolated from subventricular or hippocampal brain regions. In case of humans, the term "adult" preferably refers to an individual at an age of at least about 16 years, e.g., about 16 years to about 45 years, more preferably at least about 18 years, e.g., about 18 years to about 30 years. However, also individuals at an age of less than 16 years or greater than 45 years may be suitable donors.

NPCs were successfully isolated from various parts of brain. NPCs are abundant in fetal (embryonic) brain tissue. Preferably, NPCs are isolated from fetal midbrain tissue. Most efficiently, NPCs are prepared from human fetal brain tissue, 3 - 25 weeks of gestation, preferably 5 - 11 weeks of gestation.

Isolation of NPCs from human fetal brain tissue has been described e.g. in Buc-Caron, Neuroepithelial progenitor cells explanted from human fetal brain proliferate and differentiate in vitro, Neurobiol Dis, 2:37-47, 1995; Svendsen CN et al., Long-Term Survival of Human Central Nervous System Progenitor Cells Transplanted into a Rat Model of Parkinson's Disease, Exp Neurol 148:135-146, 1997; Sah et al, Biopotent progenitor cell lines from the human CNS , Nat Biotechnol 15:574-580, 1997; Chalmers-Redman et al, In vitro propagation and inducible differentiation of multi potential progenitor cells from human fetal brain, Neuroscience 76:1121-1128, 1997; Schwarz SC et al. Parkinsonism Relat Disord. 12(5):302-308, 2006. The disclosure of these documents is incorporated herein by reference. The technique of preparation of brain tissue and isolation of NPCs can be adapted from these protocols.

It is also possible that the NPCs are generated from other stem cells, e.g. mesenchymal stem cells or embryonic stem cells. The mesenchymal cells can be isolated from bone marrow, preferably adult bone marrow, more preferably human adult bone marrow (e.g. bone marrow stromal cells). Alternatively, the mesenchymal stem cells can be isolated from umbilical cord blood, preferably human umbilical cord blood, or from adipose tissue. Methods of isolating mesenchymal stem cells from various sources are known to those skilled in the art. They can be converted to NPCs by methods known in the art (see e.g. WO 2005/017132 A1 filed by NeuroProgen GmbH Leipzig, and references cited therein; Hermann et al. J Cell Sci. 117(Pt19):4411-4422, 2004). Using these sources of stem cells, it is possible to use autologous cells in transplantation by isolating stem cells from the body of an individual (e.g. a person suffering from Parkinson's Disease), converting the stem cells into NPCs, culturing the NPCs as described in this application, and transplanting the autologous cells into the same individual. This embodiment has the great advantage that immunological complications such as rejection of the grafted cells can be avoided.

"Neuronal cells" are cells that express the marker protein microtubule-associated protein-2 (MAP-2). They may further express the marker proteins neurofilament, calbindin and tau. The term "neurons" as used herein denotes post-mitotic neuronal cells that exhibit characteristic electrophysiological properties as described hereinafter. These consist of typical fast sodium currents and large potassium currents following depolarization. In addition, one can measure ligand gated ion currents (e. g. GABA, glutamate, acetylcholine) depending on the neuronal subtype. Neurons do not express the markers nestin and double cortin.

The term "dopaminergic cells" designates cells that are characterized by expression of the marker protein tyrosine hydroxylase (TH). Other markers characteristic of dopaminergic cells include dopamine transporter (DAT) and EN1 (engrailed homolog 1). With respect to electrophysiology, completely differentiated dopaminergic neurons typically show hyperpolarization-activated action currents (lh) (See also Storch A et al. J Chem Neuroanat. 26(2):133-142, 2003, the disclosure of which is incorporated herein by reference).

Astrocytes express the marker "glial fibrillary acidic protein" (GFAP), whereas oligodendrocytes express galactocerebrosidase (GalC).

The method according to the present invention may further comprise the steps of isolating the NPCs from a suitable source (see supra), dissociating the NPCs into single cell suspension and/or propagating the NPCs in a suitable culture medium. These steps may be carried out prior to culturing the NPCs in the presence of at least one uracil nucleotide. Suitable culture media for culturing NPCs include but are not limited to DMEM/F12 and are described infra in the Examples.

In a particular embodiment of the invention, the NPCs are cultured under an atmosphere having an oxygen concentration of less than 20% (v/v). Preferably, the oxygen concentration is less than 15% (v/v), more preferably less than 10% (v/v), still more preferably less than 5% (v/v), most preferably about 3% (v/v). The minimum oxygen concentration may be 0.1 % (v/v) or 1% (v/v). The cells may be cultured under an atmosphere of 5% (v/v) CO₂, 92% (v/v) N₂ and 3±2 % (v/v) O₂. The culturing under an reduced oxygen may be performed at any time of the culturing, for example when proliferating and/or when differentiating the NPCs as described herein.

According to a specific embodiment of the method of the invention the culture medium contains an effective Ca²⁺ concentration of less than 1.0 mmol/l, preferably less than 0.5 mmol/l, more preferably less than 0.1 mmol/l. Preferably, the total concentration of Ca²⁺ ions in the culture medium is identical to the effective concentration. If needed, a masking of Ca ions through suitable masking agents which decrease the concentration of free Ca ions may occur. For this purpose, chelating agents like EGTA, EDTA, Kronenether and others may be used. If desired, the culture medium may be free of Ca²⁺ ions apart from unavoidable contaminations; preferably, the medium is not Ca²⁺ free. A minimum amount of Ca²⁺ ions of 0.001 to 0.1 mmol/l, particularly 0.01 or 0.05 to 0.1 mmol/l culture medium, may be employed. Furthermore, the culture medium may comprise Mg ions in only low concentration, or it is free of Mg ions, apart from unavoidable contaminations. The Mg concentration of the culture medium can be less than 2 mmol/I culture medium, preferably less than 0.6 mmol/I or less than 0.1 mmol/I culture medium (See WO03/010304 A2 by NeuroProgen Leipzig GmbH). The indicated calcium ion concentrations may be present at any time of the culturing, for example when proliferating and/or when differentiating the NPCs as described herein.

It is further preferred that the NPCs are cultured at a temperature of about 35-39°C, more preferably at about 36-38°C, most preferably at about 37°C.

The term "uracil nucleotide" as used herein includes uridine 5'-triphosphate (UTP), uridine 5'-diphosphate (UDP) and uridine monophosphate (UMP). UTP and UDP are the preferred uracil nucleotides in the present invention.

The concentration of the uracil nucleotide in the culture medium may range from about 0.001 µM to about 500 µM, preferably from about 0.01 µM to about 200 µM, more preferably from about 0.1 µM to about 150 µM. According to a specific embodiment, the preferred concentration range is from about 0.01 to about 5 µM, more preferably from about 0.1 to about 3 µM, most preferably from about 0.5 to about 2 µM, e.g. about 1 µM. In another embodiment, the preferred range is from about 50 to about 150 µM, e.g. about 100 µM.

The concentration of dexamethasone in the culture medium may range from about 0.001 pM to about 500 µM, preferably from about 0.01 pM to about 200 µM, more preferably from about 0.1 pM to about 150 µM. According to a specific embodiment, the preferred concentration range is from about 0.01 nM to about 10 µM, more preferably from about 0.1 nM to about 3 µM, most preferably from about 0.1 nM to about 1 µM, e.g. about 1 nM.

The period of time during which the NPCs and the uracil nucleotide are contacted is not particularly limited. It may range from 1 minute or less to several years. Preferred periods of time are from 1 hour to 1 year, more preferably from 24 hours to 20 weeks, more preferably from 5 to 30 days wherein durations between 1 week and 2 weeks are most preferred.

The uracil nucleotide may be applied at the beginning of the culturing period or added later on after some culturing without uracil nucleotide. The uracil nucleotide may be renewed once a week in combination with a medium change. In yet another embodiment of the present invention the uracil nucleotide is supplied daily during the contacting period wherein supplementation of five consecutive days from the beginning of the contacting period is particularly preferred.

In a further preferred embodiment of the present invention the culturing method comprises the proliferation of the NPCs. According to the present invention proliferation is to be understood as a process of reproduction and/or division of cells. Therefore proliferation is the expansion of a population of cells by the continuous division of single cells into two identical daughter cells.

When proliferation of the NPCs is to be achieved the concentration of the uracil nucleotide in the culture medium is preferably from about 0.01 to about 5 µM, more preferably from about 0.1 to about 3 µM, most preferably from about 0.5 to about 2 µM, e.g. about 1 µM. The uracil nucleotide for proliferation is preferably UTP. It is also possible, however, to use UDP for proliferation.

The concentration of dexamethasone for proliferation of NPCs may be as indicated above.

It is preferred to add one or more suitable growth factor(s) to the culture medium during the proliferation of the NPCs.

Generally, growth factors in the sense of the present invention are proteins or peptides that are capable of stimulating cell division and/or differentiation. Most growth factors are proteins. When the growth factor is produced in a laboratory the growth factor has preferably the biological activity of a native mammalian growth factor which means having at least one activity of a native mammalian growth factor such as binding to the same receptor as a particular native mammalian growth factors and/or eliciting proliferation and/or differentiation and/or changes in cell size. Preferably the laboratory growth factor binds to the same receptor as a particular native mammalian growth factor. The term growth factor encompasses analogs which are deletional or substitutional mutants of a native mammalian growth factor. Furthermore, the term growth factor encompasses the growth factor from other species and naturally occurring in synthetic variants thereof. Exemplary growth factors include platelet-derived growth factor (PDGF), epidermal growth factor (EGF), vascular endothelial growth factor (VEGF), erytrhopoeitin, insulin-like growth facor-1 and -2 (IGF-1, IGF-2), transforming growth factors (TGF; TGF;), acidic and basic fibroblast growth factors (a-FGF/FGF-2, b-FGF/FGF-2), Interleukins 1,2,6 and 8 (IL-1, IL-2, IL-6, IL-8), nerve growth factor (NGF), brain-derived neurotrophic factor (BDNF), neurotrophin-3 (NT-3), interleukin-3, hematopoietic colony stimulating factors (CSFs), amphiregulin, interferon- (INF-), thyrotropin releasing hormone (TRH), and prolactin. EGF is particularly known as a 6045 Da protein with 53 amino acid residues.

Most preferred for proliferation are EGF, FGF-2 and combinations thereof.

It should be noted that also variants analogs of these agents (growth factors) which share a substantial identity with a native mammalian growth factor as listed above and which are capable of binding to receptors of the native mammalian growth factor are referred to as growth factors in the sense of the present invention. For example EGF variants or analogs which share a substantial identity with a native mammalian EGF and are capable of binding to receptor of a native mammalian growth factor can be used in the present application. These EGF variants and analogs include but are not limited to the recombinant and modified EGF having a deletion of the two C-terminal amino acids and a neutral amino acid substitution at position 51 such as asparagine, glutamine, serine or alanine (particularly EGF51N or EGF51Q) having asparagine (N) or glutamine (Q) at position 51 respectively; WO 03/040310); the EGF mutein (EGF-X16) in which the His residue at position 16 is replaced with a neutral or acidic amino acid (US Patent No. 6,191,106); the 52-amino acid deletion mutant of EGF which lacks the amino terminal residue of the native EGF (EGF-D); the EGF deletion mutant in which the amino terminal residue as well as the two C-terminal residues (Arg-Leu) are deleted (EGF-B); the EGF-D in which the Met residue at position 21 is oxidized (EGF-C); the EGF-B in which the Met residue at position 21 is oxidized (EGF-A); heparin-binding EGF-like growth factor (HG-EGF); betacellulin; amphiregulin; neuregulin; or a fusion protein comprising any of the above. Other EGF analogs or variants are described in WO 03/040310 and US Patent Nos. 6,191,106 and 5,547,935.

Fibroblast growth factor (FGF) is a family of proteins that are involved in proliferation and differentiation of several cell types. It binds to receptors belonging to the tyrosine kinase family. FGF-2 is the prototype of the fibroblast growth factor family which currently has up to 18 members of structurally related proteins. It acts as a mitogen or proliferative signal for several cell types including fibroblasts smooth muscle cells and endothelial cells and is also involved in the angiogenic process and in the formation of mesenchyme.

According to the present invention for proliferation of the NPCs, EGF and FGF-2 are particularly preferred growth factors.

EGF and FGF-2 are commercially available.
According to the present invention the growth factor(s) are preferably present in a concentration in the culture medium of from 0.2 ng/ml to 2 µg/ml, more preferably 2 ng/ml to 200 ng/ml, still more preferably 10 ng/ml to 100 ng/ml, most preferably about 20 ng/ml. The concentration of FGF-2 in the culture medium is preferably 0.2 ng/ml to 2 µg/ml, more preferably 2 ng/ml to 200 ng/ml, still more preferably 10 ng/ml to 100 ng/ml, most preferably about 20 ng/ml. The concentration of EGF in the culture medium is preferably 0.2 ng/ml to 2 µg/ml, more preferably 2 ng/ml to 200 ng/ml, still more preferably 10 ng/ml to 100 ng/ml, most preferably about 20 ng/ml.

The NPCs may be cultured in the presence of EGF and FGF-2 for at least 2 days, preferably for at least 10 days, most preferably for at least 14 days, e.g., for 20 to 70 days. In one aspect, the NPCs are cultured in the presence of EGF and FGF-2 until neurosphere formation is observed, e.g., for 10 to 20 days. These neurospheres may be further expanded in medium containing EGF and FGF-2 for 1 to 15 days, preferably for 2 to 10 days. It is also possible, however, to culture the cells in the forms of neurospheres over longer periods of time, e.g. from 1 to 50 weeks or from 2 to 10 weeks. The NPCs may first be cultured in the form of neurospheres for a few days (e.g. for 1, 2, 3, 4, 5, 6 or 7 days). This means the cells are kept in suspension culture. This suspension culture of neurospheres may be followed by a so-called two-dimensional culture, i.e. an adherent culture wherein the cells adhere to the substrate. The adherent culture may be maintained for longer periods of time.

In a particularly preferred embodiment of the present invention the proliferation of the NPCs in the presence of the uracil nucleotide has a duration of from 1 day to 10 weeks, more preferably from 5 days to 5 weeks, and most preferred of from 1 week to 3 weeks, e.g. 2 weeks. The growth factor(s) may be applied to the culture medium at the beginning of the proliferation period. In another preferred embodiment the growth factor is applied together with the uracil nucleotide but it is also possible to apply the growth factor prior to or after the uracil nucleotide application. It is also possible to first proliferate the NPCs in the presence of the one or more uracil nucleotide(s) and suitable growth factor(s), and then withdraw the uracil nucleotide(s) without withdrawing the growth factor(s). The effect of the uracil nucleotide(s) may persist for many weeks, even after removal from the culture medium.

The NPCs may be cultured in the presence of the uracil nucleotide(s) to increase the number of living cells by at least 20%, more preferably by at least 30%, still more preferably by at least 40%, most preferably by at least 50%, relative to controls (i.e. relative to NPCs cultured under the same conditions except that said uracil nucleotide(s) is/are not present). In another embodiment, the invention relates to the *in vitro*-use of at least one uracil nucleotide for increasing the number of living cells, e. g. by at least 20%, more preferably by at least 30%, still more preferably by at least 40%, most preferably by at least 50%, relative to controls (i.e. relative to NPCs cultured under the same conditions except that said uracil nucleotide(s) is/are not present).

The NPCs may be cultured in the presence of the uracil nucleotide(s) to increase the expression of the proliferation marker PCNA by at least 50%, preferably by at least 100%, more preferably by at least 150%, still more preferably by at least 200%, relative to controls (i.e. relative to NPCs cultured under the same conditions except that said uracil nucleotide(s) is/are not present). In another embodiment, the invention relates to the *in vitro*-use of at least one uracil nucleotide for increasing the expression of the proliferation marker PCNA, e. g. by at least 50%, preferably by at least 100%, more preferably by at least 150%, still more preferably by at least 200%, relative to controls (i.e. relative to NPCs cultured under the same conditions except that said uracil nucleotide(s) is/are not present). Measurement of PCNA expression can be performed as described in the examples infra.

The NPCs may be cultured in the presence of the uracil nucleotide(s) to increase the number of cells that are positive for the proliferation marker Ki67 by at least 20%, preferably by at least 40%, more preferably by at least 60%, still more preferably by at least 80% or at least 90%, relative to controls (i.e. relative to NPCs cultured under the same conditions except that said uracil nucleotide(s) is/are not present). In another embodiment, the invention relates to the *in vitro*-use of at least one uracil nucleotide for increasing the number of cells that are positive for the proliferation marker Ki67, e. g. by at least 20%, preferably by at least 40%, more preferably by at least 60%, still more preferably by at least 80% or at least 90%, relative to controls (i.e. relative to NPCs cultured under the same conditions except that said uracil nucleotide(s) is/are not present). The determination of the number of cells that are positive for Ki67 can be performed as described in the examples infra.

The NPCs may be cultured in the presence of the uracil nucleotide(s) to increase the number of cells that are positive for the stem cell marker Nestin by at least 20%, preferably by at least 40%, more preferably by at least 60%, still more preferably by at least 80%, relative to controls (i.e. relative to NPCs cultured under the same conditions except that said uracil nucleotide(s) is/are not present). In another embodiment, the invention relates to the *in vitro-*use of at least one uracil nucleotide for increasing the number of cells that are positive for the stem cell marker Nestin, e. g. by at least 20%, preferably by at least 40%, more preferably by at least 60%, still more preferably by at least 80%, relative to controls (i.e. relative to NPCs cultured under the same conditions except that said uracil nucleotide(s) is/are not present).

The determination of the number of cells that are positive for Nestin can be performed as described in the examples infra.

The NPCs may be cultured in the presence of the uracil nucleotide(s) to increase the number of cells that are positive for both Ki67 and Nestin by at least 20%, preferably by at least 40%, more preferably by at least 60%, still more preferably by at least 80% or at least 90%, relative to controls (i.e. relative to NPCs cultured under the same conditions except that said uracil nucleotide(s) is/are not present). In another embodiment, the invention relates to the *in vitro*-use of at least one uracil nucleotide for increasing the number of cells that are positive for both Ki67 and Nestin, e. g. by at least 20%, preferably by at least 40%, more preferably by at least 60%, still more preferably by at least 80% or at least 90%, relative to controls (i.e. relative to NPCs cultured under the same conditions except that said uracil nucleotide(s) is/are not present).

The NPCs may be cultured in the presence of the uracil nucleotide(s) to increase the number of cells that are positive for Nestin and negative for Ki67 by at least 20%, preferably by at least 40%, more preferably by at least 60%, still more preferably by at least 80%, relative to controls (i.e. relative to NPCs cultured under the same conditions except that said uracil nucleotide(s) is/are not present). In another embodiment, the invention relates to the *in vitro-*use of at least one uracil nucleotide for increasing the number of cells that are positive for Nestin and negative for Ki67, e. g. by at least 20%, preferably by at least 40%, more preferably by at least 60%, still more preferably by at least 80%, relative to controls (i.e. relative to NPCs cultured under the same conditions except that said uracil nucleotide(s) is/are not present).
The determination of the number of cells that are positive for Nestin can be performed as described in the examples infra.

By the method of the invention, it can be achieved that the NPCs express one, two, three or all of the following marker molecules: engrailed-1, Nurr1, sox-6 and ptx-3. Preferably, the NPCs obtained express Nurr1. More preferably, they express Nurr1 and engrailed-1.

Generally, the expression of a marker protein can be determined by methods known in the art, for example by RT-PCR or by immunocytochemistry using suitable antibodies.

In another preferred embodiment of the present invention the culturing method comprises differentiation of the NPCs. In the sense of the present invention differentiation is to be understood as a process whereby an unspecialized cell acquires the features of a specialized cell such as a neural cell. It is possible to manipulate differentiation ("directed differentiation") in that the stem cell culture conditions are optimized. Therefore differentiation is the development of cells with specialized structure and function from unspecialized precursor cells and - in general - differentiation of cells leads to a decrease in proliferation.

Differentiation according to the present invention is preferably differentiation into dopaminergic cells and/or into neuronal cells. Differentiation is usually induced by replacement of expansion media with mitogen-free media containing serum, e.g. 1% FCS, optionally supplemented by forskoline, e.g. at a concentration of about 5 µM, or by neuropathiazol, e.g. at a concentration of about 10 µM. It is particularly preferred that the culture medium for differentiation contains at least one substance chosen from the group of IL-1 (e.g. IL-1β), IL-11, LIF and GDNF (for details and suitable concentrations see WO 00/78931 A2, the disclosure of which is incorporated herein by reference). In one aspect of the invention, at least one uracil nucleotide is added to the culture medium to differentiate the NPCs into dopaminergic neural cells.

In a specific embodiment, dexamethasone is present in the culture medium for differentiating the NPCs in addition to the uracil nucleotide(s). The concentration of dexamethasone in the culture medium may be as indicated above.

In another specific embodiment, neuropathiazol is present in the culture medium for differentiating the NPCs. The concentration of neuropathiazol in the culture medium may range from about 0.1 µM to about 100 µM, preferably from about 1 µM to about 25 µM, most preferably the concentration is from about 5 µM to about 15 µM, e.g. about 10 µM.

For differentiating the NPCs a broad range of concentrations of the uracil nucleotide in the culture medium can be used. For example, the concentration of the uracil nucleotide in the culture medium may range from about 0.001 µM to about 500 µM, preferably from about 0.01 µM to about 200 µM, more preferably from about 0.5 to about 150 µM. Generally, UDP is effective already at lower concentrations of from while higher concentrations may be required when UTP is used. Accordingly, in a specific embodiment, UTP is employed as uracil nucleotide, and the preferred concentration range is from about 0.5 µM to about 150 µM, more preferably from about 1 µM to about 120 µM, e.g. about 100 µM. In another embodiment, UDP is employed as uracil nucleotide, and the preferred concentration range is from about 0.1 µM to about 50 µM, more preferably from about 0.5 µM to about 20 µM, e.g. about 1 µM to about 10 µM.

For differentiating the NPCs a broad range of concentrations of dexamethasone in the culture medium can be used. For example, the concentration of dexamethasone in the culture medium may be as indicated supra.

It is also preferred according to the present invention that the culturing method comprises proliferation as well as differentiation. In that case, proliferation and differentiation are carried out as described herein. Proliferation and differentiation can be carried out simultaneously or successively. Preferably, proliferation is carried out prior to differentiation of NPCs. It is also possible, however, to first carry out a differentiation according to the present invention, followed by a proliferation as described herein. In the latter embodiment, the differentiation is usually a partial differentiation. That means the cells are not allowed to fully differentiate into a state where they can no longer proliferate. Rather, the differentiation factor(s) are removed after a period of exposure, at a point of time when the cells are still capable of dividing but already express markers typical for dopaminergic neurons. Partial differentiation is described in detail in WO 00/78931 A2. In accordance with this invention, the duration of exposure to the differentiation factor(s) may range from about 1 hour to several days, preferably from about 2 hours to about 12 hours, e.g. from about 6 to about 8 hours.

It is further preferred, according to the present invention, that the NPCs differentiate into dopaminergic neuronal cells. It is particularly preferred that in the population of cells obtainable by the method described herein at least 5%, preferably at least 10%, more preferably at least 15%, and most preferred more than 20% of the cells are dopaminergic cells, preferably dopaminergic neuronal cells.

In the population of cells obtainable by the method of this invention, at least 10%, preferably at least 20%, more preferably at least 30%, and most preferred more than 40% of the cells may be positive for expression of the marker protein MAP-2.
In another embodiment, at least 10%, preferably at least 20%, more preferably at least 30%, and most preferred more than 40% of the cells in the population of cells obtainable by the method of this invention are positive for expression of β-tubulin III (TUJ1).

In the population of cells obtainable by the method of this invention, at least 5%, preferably at least 10%, more preferably at least 15% of the cells may be positive for expression of the marker protein TH (tyrosine hydroxylase).
In another embodiment, at least 5%, preferably at least 10%, more preferably at least 15% of the cells in the population of cells obtainable by the method of this invention are positive for expression of the marker protein dopamine transporter (DAT).
In another embodiment, at least 5%, preferably at least 10%, more preferably at least 15% of the cells in the population of cells obtainable by the method of this invention are positive for expression of the marker protein EN1 (engrailed homolog 1).
In another embodiment, at least 5%, preferably at least 10%, more preferably at least 15% of the cells in the population of cells obtainable by the method of this invention are positive for expression of all of the following marker proteins: MAP-2 and TH. In a further embodiment, the cells are further positive for one or more of the marker proteins DAT and EN1.

In yet another embodiment, the number of cells that are positive for a marker for dopaminergic cells is strongly increased by the method described herein. The method allows to achieve an increase in the number of dopaminergic cells by at least 100%, preferably by at least 200%, most preferably by at least 300%, as compared to a culture method using the same conditions except that said uracil nucleotide(s) is/are not present.

The method of the invention allows to obtain an increase in the ratio of the number dopaminergic cells to the number of neuronal cells in the population of cells. That is, the ratio of the number of cells that are positive for a marker for dopamincergic cells to the number of cells that are positive for a marker for neuronal cells is increased by the method of the invention. For example, the ratio of the number of TH-positive cells to the number of MAP-2-positive cells can be increased. The ratio of the number of dopaminergic cells to the number of neuronal cells is preferably increased at least two-fold, more preferably at least 3-fold, most preferably at least 4-fold, compared to a control culture which has been cultured under the same conditions except that no nucleotide(s) has/have been added.

The ratio of the number of TH-positive cells to the number of MAP-2-positive cells in the population of cells is usually at least 0.1, more preferably at least 0.15, still more preferably at least 0.2, most preferably at least 0.25.

It is a particular advantage of the present invention that a high number of dopaminergic cells can be generated by the in vitro culture in the presence of uracil nucleotide(s). This is a remarkable progress since it is important for transplantation purposes that a high percentage of the cells that are transplanted into a patient suffering from PD are already dopaminergic cells.

Another aspect of this invention are dopaminergic cells obtainable by the method described herein. The dopaminergic cells are characterized by the expression of one or more of the marker proteins TH, DAT, EN1, ADH 1A1, Neuregulin-1, and Nurr1. Preferably, the dopaminergic cells express at least the marker protein TH. They do usually not express the markers nestin and double cortin.

The invention further relates to a population of cells obtainable or obtained by a method desribed herein.

In a particular embodiment, the cells of the invention are not derived from embryonic stem cells. In another embodiment, the cells of the invention have not been genetically modified.

Another aspect of the invention is a population of cells as defined supra. The population of cells can be obtained by the methods described herein.

Preferably, the dopaminergic cells of the invention are isolated cells.

Another aspect of the present invention is the use of the dopaminergic cells of the invention for the manufacture of a pharmaceutical composition for treating a neurodegenerative disease and/or neuronal injury. A pharmaceutical composition in the sense of the present invention is any composition which is suitable for pharmaceutical purposes and includes compositions for transplantation purposes. Compositions for transplantation purposes are particularly preferred.

A neurodegenerative disease in the sense of the present invention is a condition which affects brain function. Neurodegenerative diseases result from deterioration of neurons. They are divided into two groups: conditions causing problems with movement and conditions affecting memory and conditions related to dementia. They are usually marked by the loss of nerve cells. Examples of neurodegenerative diseases are Alzheimer disease, Creutzfeld-Jakob disease, multiple system atrophy (MSA) and Parkinson's disease.

Neuronal injury in the sense of the present invention is to be understood as for example occurring after brain-surgical operations.

According to the present invention the use of neural cells and/or UTP and in particular the pharmaceutical composition resulting therefrom is particularly suitable for treating neurodegenerative diseases which show a predominant loss of dopaminergic neurons such as the Parkinson's disease. Parkinson's disease (PD) is a chronic disease of the central nervous system caused by a lowered level of the inhibitory neurotransmitter dopamine. PD is a slowly progressive disease that affects a small area of cells in the midbrain known as the substantia nigra. The substantia nigra is an area in the basal ganglia of the brain. Gradual degeneration of these cells causes a reduction in the vital chemical dopamine. This decrease in dopamine is what causes the symptoms of the disease. Typical symptoms include muscular tremors and weakness as well as stiffness or rigidity of the limbs and trunk, slowness of movement, postural instability or impaired balance and coordination.

Another embodiment of the present invention is the use of at least one uracil nucleotide for the proliferation and/or differentiation of NPCs. Yet another embodiment of the invention is the use of dexamethasone for the proliferation and/or differentiation of NPCs. Proliferation and differentiation are to be understood as described hereinbefore.

### Figure 1:

**P2 receptor expression and effects of UTP on hmNPCs long-term proliferation.** (A) P2Y_{1,2,4,6} and P2X₄ mRNA expression as determined by RT-PCR with the LightCycler® system. M - marker (100 bp); 1 - positive control (primary human midbrain culture); 2 - proliferating cells; 3 - differentiating cells. (B) Performance of proliferating hmNPCs grown without mitogens (EGF, FGF-2) in the presence or absence of 1 µM UTP or grown in standard media with mitogens (custom media). (C) Number of living cells and protein content 2 weeks after expansion in media containing varying UTP concentrations (0-100 µM). (D) The protein expression of the proliferation marker PCNA and of the survival marker Bcl-2 protein expression was evaluated 2 weeks following UTP treatment. (E) Representative Western blot showing the expression of PCNA and Bcl-2 two weeks following application of 1 µM UTP, or on pre-treatment with 100 µM PPDAS (UTP + PPADS). (F) Densitometric analysis of Western blots showing relative band intensities normalized to untreated samples. All data are expressed as mean ± SEM (n = 4). **P* < 0.05 vs. control cells, ***P* < 0.05 compared to UTP samples.

### Figure 2

**Effects of UTP and adenine nucleotides on hmNPCs short-term proliferation.** Human mNPCs were treated consequently for 5 d with various P2Y agonists (1 µM UTP, 100 µM ATP, 100 µM ADP, 100 µM ADβS) and immunostained for the proliferation marker Ki67 (green fluorescence) and the neural stem cell marker nestin (red fluorescence). Note an increased number of Nestin/Ki67 double-reactive NPCs following treatment with 1 µM UTP (n = 3). Scale bar: 50 µm

### Figure 3

**UTP and UDP improve dopaminergic differentiation of hmNPCs.** (A) Double staining of dopaminergic neurons. After 2 weeks of expansion followed by 1 week of differentiation in the presence of 100 µM UTP, the contribution of TH-immunopositive cells (red) to the total of Tuj1-immunopositive cells (green) is increased. (B) Percentages of TH-positive hmNPCs normalized to control obtained by immunostaining of hmNPCs differentiated in UTP-containing media. (C) hmNPCs were treated during proliferation (2 weeks) and finally differentiated for 1 week. TH expression was assessed in parallel by Western blot and immunocytochemistry. (D) Densitometry of TH-positive bands obtained by Western blotting following incubation of hmNPCs with 1 µM UTP during expansion, with application of 100 µM UTP during the differentiation period. UTP-treated samples were run in parallel with the same sample pre-treated with 100 µM PPADS. (E) UDP treatment of hmNPCs during the differentiation period showing TH expression as revealed by immunoblotting. **P* < 0.05 vs. untreated hmNPCs (n = 3). Scale bar = 50 µm.

### Figure 4

**UTP induces phosphorylation of ERK1/2 in hmNPCs.** (A) Human mNPCs were expanded in mitogen-free medium for 48 hours. The MEK inhibitor U0126 (10 µM) was added to the medium 15 min before UTP (1 µM) and the cells were incubated for 30 min prior to harvesting and lysing in the PhosphoSafe® extraction buffer. Detection of bands was performed by Western blot. The amount of the phosphorylated ERK1/2 (p-ERK1/2), normalized to total ERK1/2 is shown. (B) Differentiation media for hmNPCs was supplemented once with 100 µM UTP or with UTP following pre-treatment with 10 µM suramin or MEK inhibitor (10 µM). Proteins were extracted after one week and processed for the estimation of TH expression and pERK1/2 expression. The Figure shows a representative Western blot (top) and the quantification (bottom). **P* < 0.05 vs. untreated hmNPCs (n = 3).

### Figure 5

**Antagonism of ADP on hmNPC proliferation and dopaminergic differentiation.** (A) Number of living cells as determined after 2 weeks of incubation in expansion media supplemented with ADP (100 µM), UTP (100 µM) or both. On coapplication, ADP was added 30 min before UTP. (B) Number of TH-positive cells as determined by immunocytochemical staining for the dopaminergic marker TH, counting 8 randomly selected fields. The cells were once treated with the nucleotides (100 µM ADP, 100 µM UTP or both) only during the differentiation period of one week. **P* < 0.05 (n = 3) vs. untreated hmNPCs.

### Figure 6

Effects of dexamethasone on proliferation of NPCs.

### Figure 7

Effects of dexamethasone on differentiation of NPCs into dopaminergic cells.

The invention is illustrated on the basis of the following non-limiting examples and figures:

### Example 1: Uracil nucleotides stimulated neural precursor cell proliferation and dopaminergic differentiation

### Material and methods

### Preparation of human midbrain-derived NPCs (hmNPCs)

Human fetal midbrain tissue was used to generate hmNPCs cultures. Samples were harvested and supplied by Advanced Bioscience Resources Inc., Alameda, CA, according to NIH and local IRB guidelines. Only if there was no evidence for fetal pathology and informed consent was obtained, parts of the brain were transferred to our laboratory. The quality of the tissue was estimated by FACS analysis (dopamine transporter - DAT expression analysis, when more than 0.5% cells positive for DAT it was considered as mesencephalic) and this was confirmed by tyrosine hydroxylase (TH)- immunoreactivity. Primary cultures were analyzed by RT-PCR for markers of dopaminergic neurons (TH, DAT and EN1).

Tissue from a 10-14-week-old fetus was dissociated into single-cell suspension by incubating in 0.1 mg/ml papain / DNase solution (100 µg/ml; Roche, Mannheim, Germany) for 30 min at 37°C, followed by washing with PBS, incubation in antipain (50 µg/ml; Roche) for 30 min at 37°C, and finally homogenization by gentle trituration using fire-polished Pasteur pipettes. Propagation of hmNPCs was performed in a monolayer via plating onto polyornithine-fibronectin pre-coated culture dishes at a density of 30,000 cells / cm². For expansion of hmNPC defined media (DMEM/F12) without any xenogenic material (e.g. serum supplements, serum, etc.) was used, but with addition of epidermal growth factor (EGF) and fibroblast growth factor2 (FGF-2; 20 ng/ml each, both from PromoCell, Heidelberg, Germany). Cells were expanded for prolonged periods (> 10 passages) in reduced atmospheric oxygen (3 %) (Storch et al. 2001; Milosevic et al. 2005).

### Experimental design

To determine long-term proliferation, hmNPCs were plated and expanded for 2 passages with or without addition of various agents (see below) to the custom expansion media containing the growth factors EGF and FGF2. Different fetal midbrain tissues were included in this study. At the end of the proliferation period, differentiation of hmNPCs was induced via replacement of expansion media with mitogen-free media containing 1% FCS, 5 µM forskoline (Sigma-Aldrich Chemie GmbH Munich, Germany), and 100 pg/ml IL-1β (Sigma). The differentiation and expansion media were supplemented with different drugs as appropriate. Prior to immunocytochemistry or protein extraction, hmNPCs were allowed to differentiate for 1 week. To determine short-term proliferation via Ki67 immunostaining, the cells were seeded into 24-well plates (70,000 cells/well) and left overnight to rest. P2 receptor agonists (ATP, UTP, ATP + UTP, ADP, ADPβS) were supplied daily for 5 consecutive days. Ki67/nestin double-positive cells were counted in 8 randomly selected fields of nucleotide-treated or untreated samples. For the assessment of MAPK phosphorylation the cells were kept free of mitogens for 2 d prior to addition of the MEK-inhibitor in expansion media. In addition, the MEK-inhibitor was added during the differentiation period 15 min before UTP administration.

### Drug treatment

UTP, UDP, ATP, ADP, ADPβS and suramin were purchased from Sigma (Sigma-Aldrich Chemie GmbH Munich, Germany). UTP and UDP were applied at different concentrations (1 µM, 10 µM, 100 µM), ATP at 100 µM, ADP at 100 µM, and ADPβS at100 µM. During expansion of the cells, the nucleotides were renewed once a week with every media change, unless otherwise stated. They were applied once at the beginning of the differentiation period. The P2 receptor antagonist suramin (10 µM) was added to expansion media 30 min prior to addition of UTP (100 µM). The stock solution of the MEK1/2 inhibitor U0126 (Promega Corporation, Mannheim, Germany) was prepared in dimethyl sulfoxide (DMSO) at a concentration of 10 mM. The stock solution was then diluted in 0.1 M PBS to obtain a concentration of 1 mM. MEK inhibitor (final concentration 10 µM) was added to mitogen-free expansion media, 15 min before UTP treatment (1 µM) of hmNPCs.

### LightCycler RT-PCR analysis

One-step RT-PCR was performed with a LightCycler (Roche, Mannheim, Germany) in a total volume of 20 µl containing 50 ng of total RNA, 4 mM MgCl₂, 0.5 µM each primer, LightCycler RT-PCR reaction Mix SYBR Green I (1x) and LightCycler RT-PCR Enzyme Mix (Roche). The primers for human P2Y receptors were as follows: P2Y₁ primers: 5'-GAGGGCCCGGCTTGATT - 3' (SEQ ID NO:1) and 5' - ATACGTGGCATAAACCCTGTCA - 3' (SEQ ID NO:2), 67 bp product; P2Y₂ primers: 5' - TGGTGCGCTTCCTCTTCTACA - 3' (SEQ ID NO:3) and 5' - ACCGGTGCACGCTGATG - 3' (SEQ ID NO:4), 72 bp; 5' - TCATGGCTCGTCGCCTGTA - 3' (SEQ ID NO:5) and 5' - AGAGAGCGGAGGCGAGAAG - 3' (SEQ ID NO:6), 67 bp; P2Y₆ primers: 5'-CCTGCCCACAGCCATCTT - 3' (SEQ ID NO:7) and 5' - CAGTGAGAGCCATGCCATAGG - 3' (SEQ ID NO:8), 116 bp product (Koizumi et al. 2004). The primers for human P2X₄ were: 5'- TTGTGTGGGAAAAGGGCTAC - 3' (SEQ ID NO:9) and 5' - CCTGTGTCTGGTTCATGGTG - 3' (SEQ ID NO:10), 198 bp product and GAPDH primers were: 5' - GCTAGTGGTGGACCTGACCT - 3' (SEQ ID NO:11) and 5' - TGCTGTAGCCAAATTCGTTG - 3' (SEQ ID NO:12), 240 bp. Reverse transcription was performed at 48°C for 30 min. The denaturation and amplification conditions were 95°C for 30 s. Each cycle of PCR included immediate denaturation at 95°C, 20 s of primer annealing at 56°C and 15 s of extension/synthesis at 72°C followed by up to 40 cycles of PCR.

### Determination of the cell number

Cell counting of control (untreated) and treated hmNPCs was performed using a hemocytometer. Adherent cells were collected by detachment with accutase^{™} (PAA Laboratories, Pasching, Austria) for 10 min at 37°C. To measure trypan blue exclusion (cell viability), cells were incubated in triplicate in 0.25% dye solution (Sigma). The number of total and trypan blue-positive cells was then determined by counting at least 300 cells per sample in a hemocytometer.

### Determination of protein content and Western blotting

In order to prepare protein extracts, hmNPCs were collected, washed with ice-cold PBS and lysed in buffer [10 mM HEPES-KOH (pH 7.9), 10 mM KCI, 1.5 mM MgCl₂, 0.1% NP-40], supplemented with protease inhibitor cocktail (Roche). After a brief centrifugation at 4°C/10 000×g, protein concentrations and protein yield from extracts were determined using the Bradford method (Bio-Rad, Hercules, CA, USA). To preserve the phosphorylation state of proteins, extracts for the MAPK experiment were obtained using the PhosphoSafe^{™} extraction reagent containing phophatase inhibitors (Novagen). Cell lysates (50 µg) were mixed with sample loading buffer [125 mM Tris-HCl (pH 6.8), 4% (wt/vol) sodium dodecyl sulfate, 20% (vol/vol) glycerol, 200 mM dithiothreitol, 0.04% (wt/vol) bromphenol blue]. Proteins were resolved on a sodium dodecyl sulfate (8-12%) polyacrylamide gel (SDS-PAGE) and transferred to a Hybond-ECL nitrocellulose membrane (GE Healthcare, Freiburg, Germany). Membranes were blocked with 5% (wt/vol) nonfat dry milk in PBS-T (PBS, 0.1% (vol/vol) Tween 20) for 2 h at room temperature and subsequently incubated with the desired primary antibody (diluted in PBS containing 5% non-fat milk and 0.1% Tween 20) overnight at 4 °C with gentle agitation. The membrane was incubated with horseradish peroxidase-coupled secondary antibody for 1 h at room temperature. Chemiluminescence detection was performed by incubating the membranes with SuperSignal-Dura substrate (Pierce Biotechnology, Rockford, U.S.A) followed by analysis on a CCD cooling camera (Fuji LAS-1000plus). The chemiluminescence was quantified using the AIDA, two-dimensional densitometry software (Raytest lsotopenmeb, Straubenhardt, Germany). Primary antibodies used for Western blotting were as follows: mouse monoclonal anti-PCNA antibody (Santa Cruz, Heidelberg, Germany); mouse monoclonal anti-Bcl-2 antibody (Santa Cruz), mouse monoclonal anti-actin antibody (MP Biomedicals, Eschwege, Germany); rabbit polyclonal anti-β-tubulin III (anti-Tuj1) antibody (Covance, Freiburg, Germany); rabbit polyclonal anti-TH antibody (Santa Cruz, Heidelberg, Germany); rabbit anti-MAP kinase (Zymed Laboratories), anti-phospho MAP kinase (Cell Signaling Technology, Inc., Danvers, USA).

### Immunofluorescence

Expanded or one week differentiated hmNPCs were fixed with 4% paraformaldehyde in PBS for 10 min at room temperature and washed with PBS, counterstained with the DNA-binding dye 4'-6-Diamidino-2-phenylindole (DAPI, 2 µg/ml in PBS) for 15 min at room temperature, washed twice in PBS followed by incubation in blocking buffer (10% FCS, 0.2% Triton-X 100 in PBS, pH 7.2) for 30 min at room temperature. After incubation with a primary antibody (over night at 4°C in blocking buffer), the cells were incubated with fluorescent secondary antibodies, Alexa Fluor® 488 conjugate or Alexa Fluor® 594 conjugate (Molecular Probes, Eugene, USA). Cover-slips were mounted onto glass slides and examined under a fluorescence microscope (Zeiss Axiovert 200). Acquisition of the cells was performed using the Image-analysis software AxioVision 4 (Carl Zeiss AG, Jena, Germany). The following primary antibodies were used for immunofluorescence: rabbit polyclonal anti-β-tubulin III (anti-Tuj1) antibody, rabbit polyclonal anti-tyrosine hydroxylase antibody , mouse monoclonal anti-nestin antibody (Pharmingen, San Diego, CA, USA), rabbit polyclonal anti-Ki67 antigen antibody (Novocastra Laboratories Ltd, Newcastle upon Tyne, UK). Estimation of the TH-positive cell numbers was performed by counting of cells among approximately 1000 differentiated cells in total within 6 randomly selected fields.

### Cell death /cell cycle assay

Untreated (control) and treated exponentially growing hmNPCs were prepared for cell cycle analysis by lysing cells in 300 µl of hypotonic lysis buffer (0.1% sodium citrate, 0.1% Triton X-100, and 50 µg/ml propidium iodide [PI]). The cells were subsequently analyzed by flow cytometry, on a FACScan (Becton Dickinson, Heidelberg, Germany), using 488 nm excitation, gating out doublets and clumps using pulse processing and collecting fluorescence above 620 nm according to the method of Nicoletti et al. (Nicoletti et al. 1991). Histograms of DNA content were acquired using the CellQuest software. The number of nuclei present in each peak of the histogram (sub-G1, G1, S, G2/M) was analyzed by measuring the peak area. For data analysis and correction of the background, noise histograms were processed with the ModFit LT software (Verity, Turramurra, Australia).

### Electrophysiology

Patch-clamp analyses of hmNPCs was carried out after *in vitro* differentiation for 3 weeks with or without 100 µM UTP at room temperature using an inverted microscope DMIL (Leica, Bensheim, Germany) and an EPC-9 amplifier (HEKA, Lambrecht, Germany). Recordings of voltage-gated potassium and sodium channels were obtained in the whole-cell voltage-clamp mode by stepwise depolarisations with increasing amplitudes from the holding potential of - 70mV to 50mV in steps of 10mV. Ligand-gated P2X-channels were investigated in the whole-cell voltage-clamp mode by rapid application of 300 µM ADP, ATP, and UTP with a SF-77B perfusion fast-step system (Warner Instruments Inc., Hamden, USA). Series resistance values were continuously measured during all recordings. The external bath solution contained (in mM): 142 NaCl, 1 CaCl₂, 8 KCI, 6 MgCl₂, 10 Glucose, and 10 HEPES (pH 7.4; 320 mOsm). Micropipettes were formed from thin-walled borosilicate glass (Science Products, Hofheim, Germany) with a Flaming Brown electrode puller P-97 (Sutter Instrument Co., Novato, USA) and a Micro Forge (Narishige, Tokio, Japan). Electrodes had resistances of 2-4 MΩ when filled with the internal solution containing (in mM): 153 KCI, 1 MgCl2, 10 HEPES, 5 EGTA, and 2 MgATP (pH 7.3; 305 mOsm). Whole cell currents were low-pass filtered at 2-5 kHz, digitized at 10 kHz, and analyzed using PulseFit software (HEKA, Lambrecht, Germany) and Prism 4 (GraphPad Software Inc, San Diego, USA). Numerical data were expressed as mean ± SEM. Statistical significance, using Student's *t* test (two tailed, unpaired), was taken as P < 0.05.

### RNA isolation and Affymetrix arrays

Total RNA was extracted from 5x10⁶ NPCs using the commercial kit following the manufacturer's protocol (RNeasy kit, Qiagen, Hilden, Germany). RNA integrity and concentration was examined on an Agilent 2100 Bioanalyzer (Agilent Technologies, Palo Alto, CA, USA) using the RNA 6.000 LabChip Kit (Agilent Technologies) according to the manufacturer's instructions. Protocols for mRNA quality control and gene expression analysis were performed as suggested by Affymetrix. Gene expression was assayed using a chip containing oligonucleotide probes for approximately 22,000 human genes (Affymetrix Human Expression Array HG-U133A) as previously described (Milosevic et al. 2005).

### Microarray data analysis

Affymetrix GeneChip data were extracted from fluorescence intensities and were scaled in order to normalize data for inter-array comparison using the MAS 5.0 software according to the manufacturer's instructions (Affymetrix). To detect differentially regulated genes, chip comparison was performed with MAS 5.0 and the Data Mining Tool software (Affymetrix).

### Statistical analysis

Statistical analyses (one-way analysis of variance, ANOVA, followed by Tukey posthoc test) were performed using the SigmaStat software package (Jandel Corp., San Rafael, CA). Results are expressed as means ± SEM. Statistical significance was defined as P < 0.05.

### Results

### Evidence for P2 receptor expression in hmNPCs

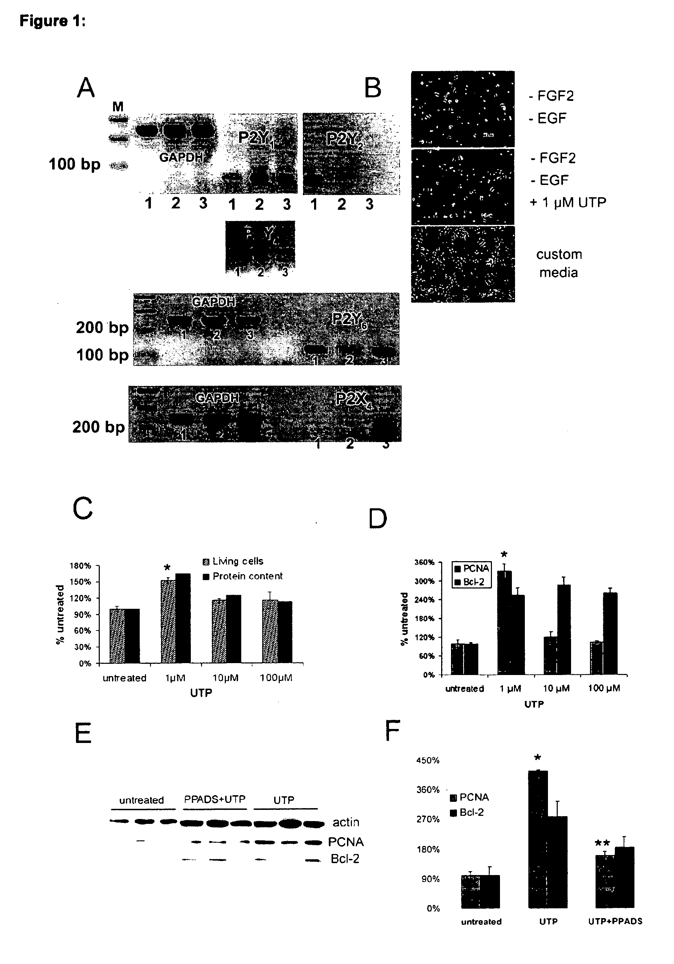

Microarray data analysis of expanded hmNPCs revealed the presence of three subtypes of nucleotide receptors only, namely P2Y₁, P2Y₄ and P2X₄ (Table 1). In addition hmNPCs expressed mRNA for several ectonucleotidases that differ in catalytic properties. These include the tissue non-specific (liver/bone/kidney) form and the placental-like form of alkaline phosphatase, ecto-nucleotide pyrophosphatase/phosphodiesterase2 (NPP2, autotaxin) and two members of the ecto-nucleoside triphosphate disphophohydrolase family (E-NTPDases) (Table 1). Whereas NTPDase1 is a cell surface-located enzyme that hydrolyzes nucleoside tri- and diphosphates about equally well, NTPDase4 resides exclusively in the Golgi apparatus (Zimmermann, 2000). In this report, we focus on P2Y receptor expression and function. Microarray data were confirmed by LightCycler RT-PCR. As shown in Figure 1A, hmNPCs express mRNAs for P2X₄, P2Y₁ and P2Y₄ receptors but not for the P2Y₂ receptor. Since the presence of ecto-nucleotidases could lead to the formation not only of extracellular ADP but also of UDP we included the UDP sensitive P2Y₆ receptor in our PCR-analysis. In spite of the negative results obtained with the microarray technology, mRNA encoding the P2Y₆ receptor was found to be expressed by hmNPCs (Fig. 1A).

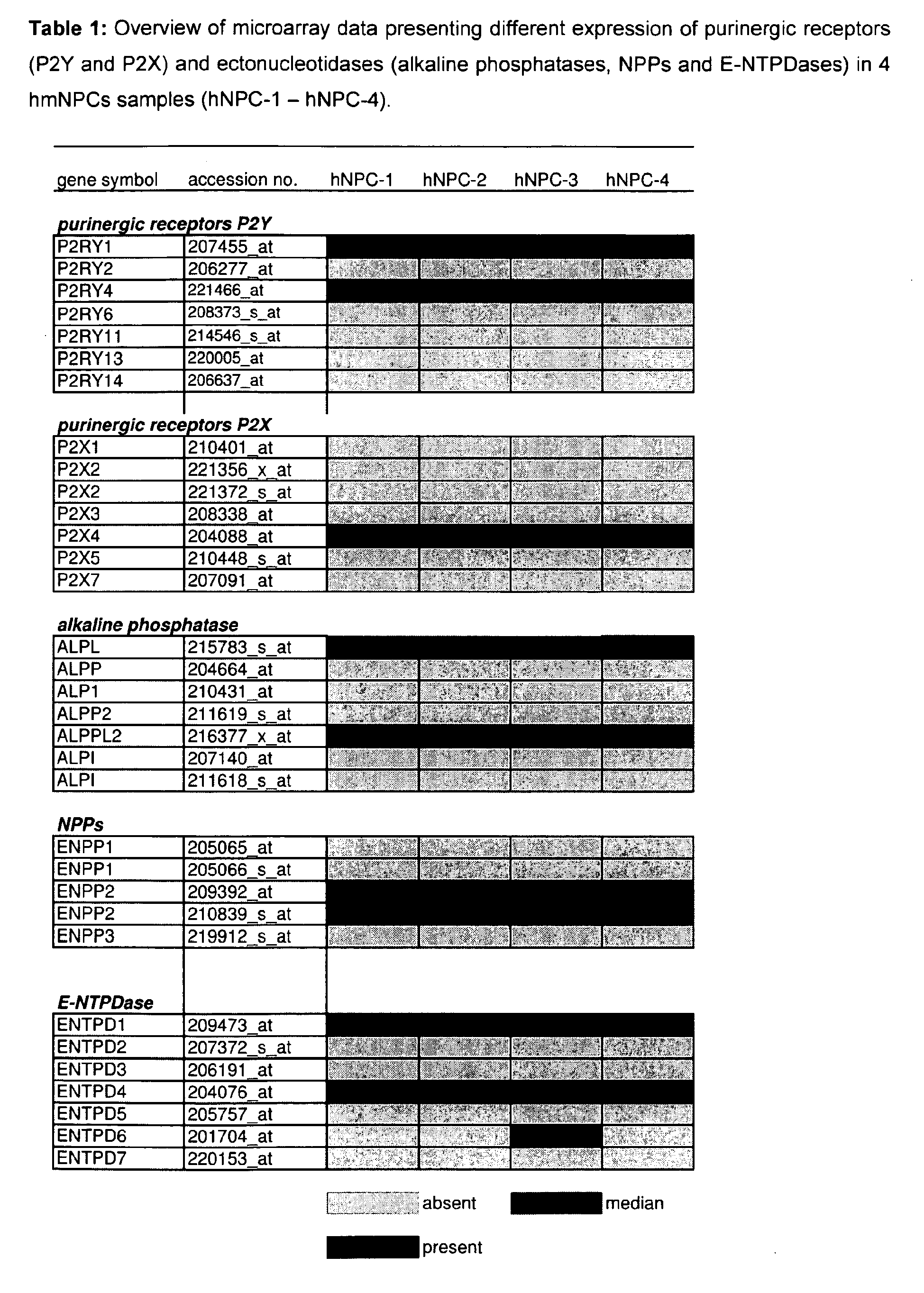

### UTP augments proliferation of hmNPCs

Human mNPCs stopped growing without EGF and FGF2, but performed slightly better when treated with 1 µM UTP once in 5 days (Figure 1B). After 2 passages (approximately 2 weeks of expansion with or without UTP), untreated (control), agonist-treated and antagonist-treated hmNPCs were counted and subjected to protein extraction. One-way analysis of variance (ANOVA) revealed a notably higher number of living cells when these were treated with UTP (n = 4; P = 0.022). Of all tree tested UTP concentrations, only 1 µM was effective in increasing the number of living cells as compared to controls (152 ± 6% vs. 100 ± 5%, respectively, Fig. 1C). When compared to untreated samples, the cells incubated with 1 µM UTP also yielded more protein than at 10 µM or 100 µM UTP (165% vs. 125% and 113%, respectively, Fig. 1C). In addition, Western blot analyses of UTP-treated NPCs revealed a significant improvement of cell proliferation (n = 4; *P* = 0.018). When compared to control cells, the expression of the proliferation marker PCNA was significantly increased only after application 1 µM UTP (330 ± 23%). However, expression of the survival marker Bcl-2 did not significantly change with increasing UTP concentrations (Fig. 1D). The preferential P2Y blocker PPADS (100 µM), supplemented 2 times during the 2 weeks proliferation period before addition of 1 µM UTP, reduced the PCNA expression to control levels (Fig. 1E,F)

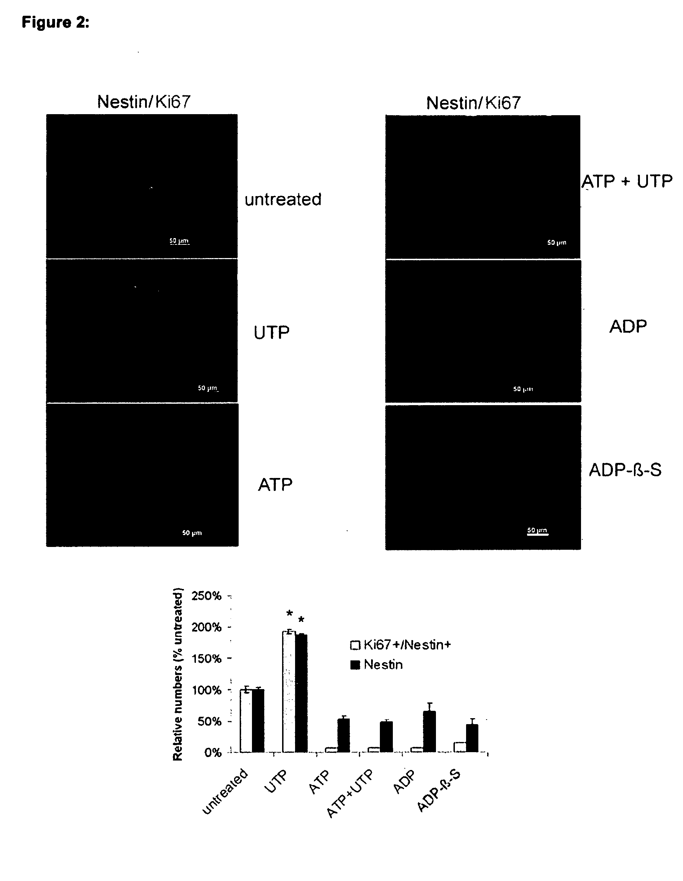

Human mesencephalic NPCs were further treated for 5 subsequent days with various P2Y agonists. As shown in Figure 2, UTP treatment markedly induced proliferation of nestin containing cells. As revealed by immunocytochemistry, 1 µM UTP increased the contribution of NPCs double-immunoreactive for the proliferation marker Ki67 and the stem cell marker nestin to 193 ± 3% (n = 3; *P* < 0.001). The same treatment lead to an increase of cells immunoreactive for nestin alone to 188 ± 2 % (Fig. 2). By contrast, administration of ATP, ADP and ADPβS exhibited anti-proliferative effects on hmNPCs, resulting in a strong reduction of Ki67-positive cells.

### UTP and UDP increase the pool of dopaminergic hmNPCs

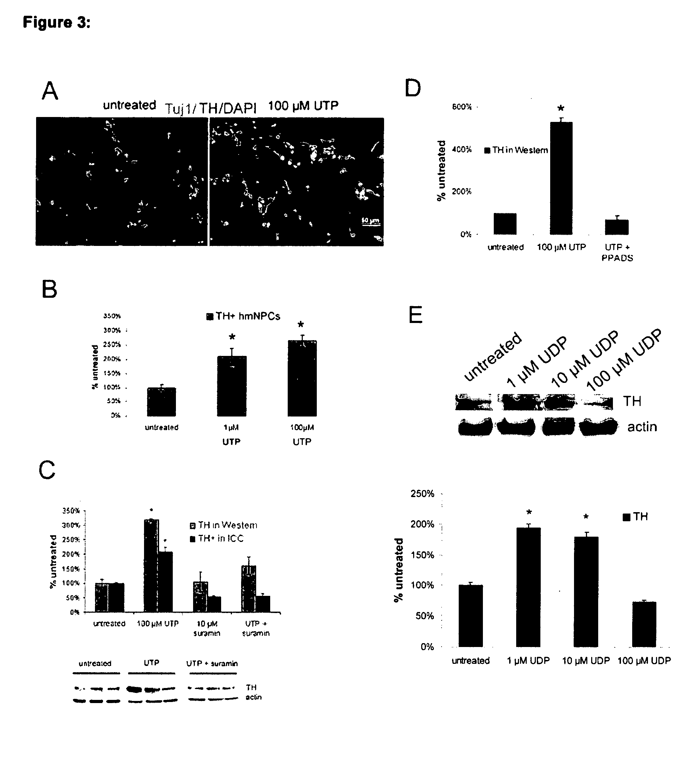

Dopaminergic differentiation was analyzed by immunocytochemical staining of the differentiated hmNPCs with an antibody directed against tyrosine hydroxylase (TH, Fig. 3A). Incubation of hmNPCs with varying concentrations of UTP during 2 weeks of expansion and one week of differentiation resulted in a significant increase in the number of the dopaminergic neurons (n = 3; *P* < 0.001). A profound increase in the number of TH-positive cells was obtained after application of 1 µM UTP (211 ± 30%). Exposure to 100 µM UTP during proliferation and differentiation, induced a further increase to 267 ± 20% (Fig. 3B). As compared to controls, TH protein expression in Western blots reached 319 ± 5% following long-term treatment with 100 µM UTP. Application of the P2 receptor antagonist suramin (10 µM) 30 min before addition of 100 µM UTP, abrogated dopaminergic differentiation as identified by a reduced number of TH-positive cells and decreased TH protein content in NPCs (Fig. 3C). The best results regarding TH protein expression were obtained when cells were first treated with 1 µM UTP during 2 weeks of expansion followed by 1 week of differentiation in the presence of 100 µM UTP. In this particular case, TH expression increased to 529 ± 20% (n = 3) and was completely blocked by pre-treatment with 100 µM PPADS (Figure 3D).

Treatment of cells with 1 µM and 10 µM UDP during the differentiation period, increased TH expression in immunoblots to 194 ± 7% (n = 3) and 180 ± 8%, respectively (Figure 3E). In contrast to UTP (Figure 3B,C) no increase in TH protein levels was observed at high (100 µM) concentrations of UDP.

### UTP induces MAPK phosphorylation in hmNPCs

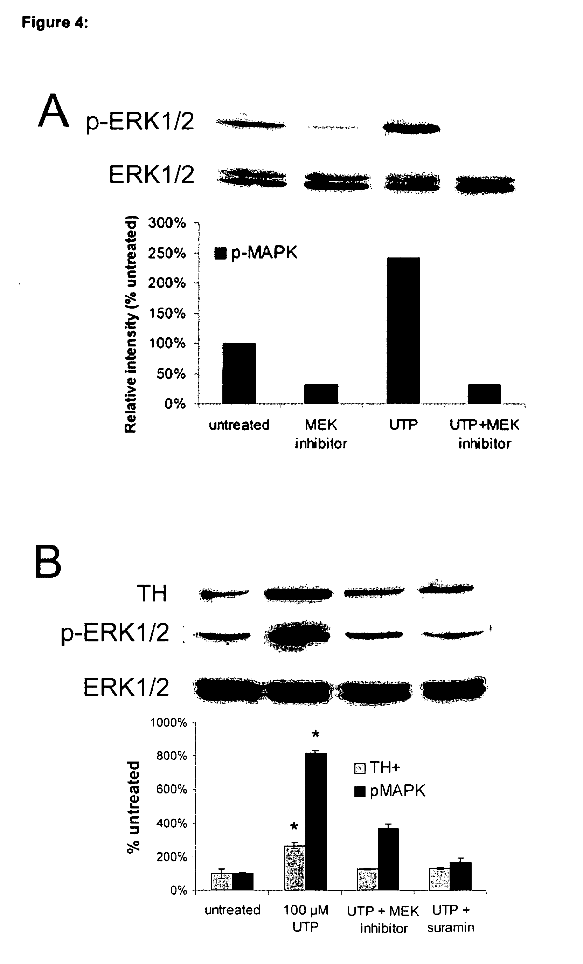

ERK1/2 (MAPK) was phosphorylated following short-term exposure (30 min) of hmNPCs to 1 µM UTP supplemented to the expansion media. Pretreatment for 15 min with 10 µM of the MEK inhibitor U0126 abrogated the UTP-induced phosphorylation of ERK1/2 (P-MAPK) (Figure 4A). To investigate the involvement of MEK-ERK signalling in the UTP-mediated potentiation of dopaminergic differentiation, hmNPCs were allowed to differentiate for one week with 100 µM UTP in the presence or absence of the MEK inhibitor U0126 (10 µM) or the P2 receptor antagonist suramin (10 µM). Protein extracts were examined for the expression of TH and phosphorylated ERK1/2. Both parameters were increased following UTP treatment. They remained at basal levels on pre-treatment with U0126 or suramin (Figure 4B). The MEK inhibitor also reduced the proliferation of hmNPCs (data not shown).

### Effect of adenine nucleotides on hmNPCs performance

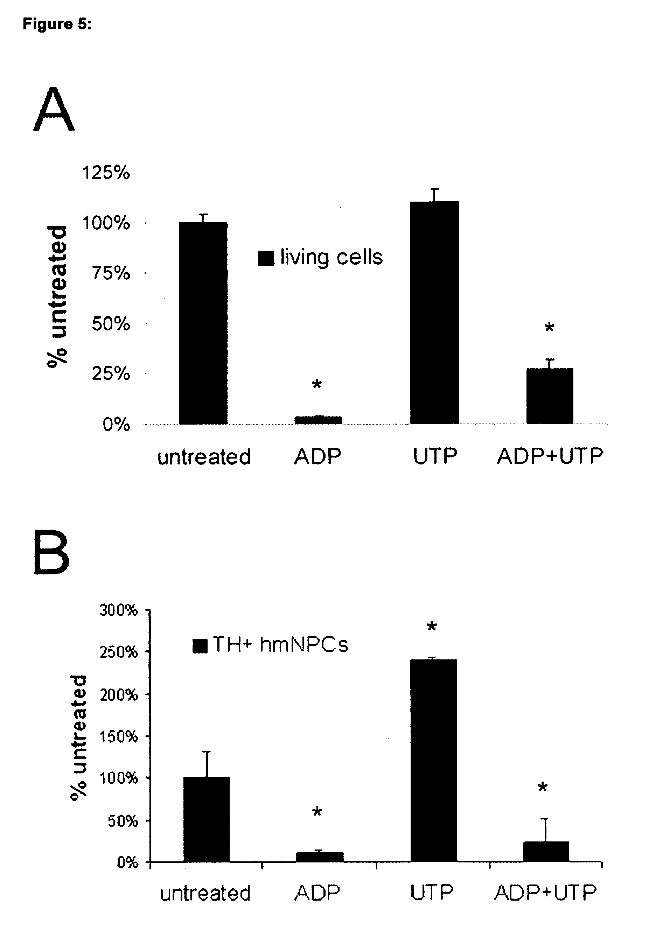

Long-term treatment of hmNPCs with ADP (100 µM), resulted in a striking decrease in the number of living cells (4 ± 0.5%; n = 3). In addition, ADP, when coapplied, blocked the effect of UTP (both 100 µM), yielding a lower number of viable cells than UTP alone (27 ± 5% vs. 110 ± 6%; Figure 5A).
When differentiation was initiated from the same number of hmNPCs expanded without nucleotides (80,000 cells plated per a glass slide), ADP (100 µM) produced clearly fewer dopaminergic cells as compared to control (11 ± 3%; n = 3). This number did not significantly differ from the combination ADP+UTP (24 ± 3%). An increase in TH-positive cells was obtained following application of 100 µM UTP (239 ± 27%, Figure 5B).
An analysis of the cell cycle distribution was performed 48 hours following treatment with various nucleotides. Late stages of apoptosis were quantified by staining of DNA contents with PI, which identified a sub-diploid peak (sub-G1) as representative of apoptotic cells. Survival of control samples was > 99%. None of the applied nucleotides induced cell death in hmNPCs after 48 hours. However, adenine nucleotides showed a tendency to influence the cell cycle and decreased the percentage of the cells in the S-phase and G2/M-phase and increased the percentage of cells in the G0/G1-phase (Table 2). 30 minutes pretreatment with 10 µM suramin or 100 µM PPADS failed to block the anti-proliferative effects of ATP, ADP and ADPβS (Table 2).

**Table 2: Cell-cycle-phase distribution of hmNPCs following exposure to various adenine nucleotides and their antagonists (suramin and PPADS). Cells were treated for 48 h with 100 µM nucleotides (ATP, ADP, ADPβS), pretreated with 10 µM suramin or 100 µM PPADS (30 minutes) and analysed by FACS (results shown are mean ± SEM, n = 3). * P < 0.05 vs. untreated.**

| **hmNPCs** | **Sub-G1** | **G0/G1** | **S** | **G2/M** |
|---|---|---|---|---|
| *untreated* | 0.59 | 80.39 ± 0.12 | 15.13 ± 0.13 | 4.49 ± 0.10 |
| | | * | * | * |
| *100 µM ATP* | 0.56 | 91.69 ± 1.19 | 6.89 ± 1.17 | 1.42 ± 0.11 |
| | | * | * | * |
| *100 µM ADP* | 0.54 | 84.64 ± 0.41 | 11.63 ± 0.2 | 3.72 ± 0.20 |
| | | * | * | |
| *100 µM ADP-B-S* | 0.82 | 84.21 ± 0.06 | 12.17 ± 0.05 | 3.61 ± 0.01 |
| | | * | * | * |
| *ATP+suramin* | 0.29 | 91.51 ± 0.16 | 6.99 ± 0.12 | 1.50 ± 0.04 |
| | | * | * | * |
| *ATP+PPADS* | 0.24 | 93.05 ± 0.29 | 4.63 ± 0.48 | 2.32 ± 0.34 |

### Electrophysiology

Electrophysiological analyses of voltage-gated channels showed no significant difference between maximal potassium and sodium current amplitudes of UTP-treated (n=15) and untreated (n=10) differentiated hmNPCs. The application of ADP, ATP, or UTP did not induce any detectable inward currents indicating that there is no expression of functional ligand-gated P2X-channels in hmNPCs. We were not able to measure any hyperpolarization-activated action currents (lh) typical for dopaminergic neurons.

### References

Arthur D. B., Akassoglou K. and Insel P. A. (2005) P2Y2 receptor activates nerve growth factor/TrkA signaling to enhance neuronal differentiation. Proc Natl Acad Sci U S A. 102, 19138-19143. Epub 12005 Dec 19119.

Baker O. J., Camden J. M., Ratchford A. M., Seye C. I., Erb L. and Weisman G. A. (2005) Differential coupling of the P2Y(1) receptor to Galpha(14) and Galpha(q/11) proteins during the development of the rat salivary gland. Arch Oral Biol 3**.**

Behrsing H. P. and Vulliet P. R. (2004) Mitogen-activated protein kinase mediates purinergic-enhanced nerve growth factor-induced neurite outgrowth in PC12 cells. J Neurosci Res. 78, 64-74.

Bogdanov Y. D., Wildman S. S., Clements M. P., King B. F. and Burnstock G. (1998) Molecular cloning and characterization of rat P2Y4 nucleotide receptor. Br J Pharmacol. 124, 428-430.

Cavaliere F., Nestola V., Amadio S., D'Ambrosi N., Angelini D. F., Sancesario G., Bernardi G. and Volonte C. (2005) The metabotropic P2Y4 receptor participates in the commitment to differentiation and cell death of human neuroblastoma SH-SY5Y cells. Neurobiol Dis. 18, 100-109.

Cheung K. K., Ryten M. and Burnstock G. (2003) Abundant and dynamic expression of G protein-coupled P2Y receptors in mammalian development. Dev Dyn. 228, 254-266.

Communi D., Motte S., Boeynaems J. M. and Pirotton S. (1996) Pharmacological characterization of the human P2Y4 receptor. Eur J Pharmacol. 317, 383-389.

D'Ambrosi N., Murra B., Vacca F. and Volonte C. (2004) Pathways of survival induced by NGF and extracellular ATP after growth factor deprivation. Prog Brain Res. 146, 93-100.

D'Ambrosi N., Murra B., Cavaliere F., Amadio S., Bernardi G., Burnstock G. and Volonte C. (2001) Interaction between ATP and nerve growth factor signalling in the survival and neuritic outgrowth from PC12 cells. Neuroscience. 108, 527-534.

Di lorio P., Kleywegt S., Ciccarelli R., Traversa U., Andrew C. M., Crocker C. E., Werstiuk E. S. and Rathbone M. P. (2002) Mechanisms of apoptosis induced by purine nucleosides in astrocytes. Glia. 38, 179-190.

Fam S. R., Paquet M., Castleberry A. M., Oller H., Lee C. J., Traynelis S. F., Smith Y., Yun C. C. and Hall R. A. (2005) P2Y1 receptor signaling is controlled by interaction with the PDZ scaffold NHERF-2. Proc Natl Acad Sci USA. 102, 8042-8047. Epub 2005 May 8018.

Franke H. and Illes P. (2006) Involvement of P2 receptors in the growth and survival of neurons in the CNS. Pharmacol Ther. 109, 297-324. Epub 2005 Aug 2015.

Harada H., Chan C. M., Loesch A., Unwin R. and Burnstock G. (2000) Induction of proliferation and apoptotic cell death via P2Y and P2X receptors, respectively, in rat glomerular mesangial cells. Kidney Int. 57, 949-958.

Kennedy C., Qi A. D., Herold C. L., Harden T. K. and Nicholas R. A. (2000) ATP, an agonist at the rat P2Y(4) receptor, is an antagonist at the human P2Y(4) receptor. Mol Pharmacol. 57, 926-931.

Koizumi S., Fujishita K., Inoue K., Shigemoto-Mogami Y. and Tsuda M. (2004) Ca2+ waves in keratinocytes are transmitted to sensory neurons: the involvement of extracellular ATP and P2Y2 receptor activation. Biochem J. 380, 329-338.

Koles L., Furst S. and IIIes P. (2005) P2X and P2Y receptors as possible targets of therapeutic manipulations in CNS illnesses. Drug News Perspect. 18, 85-101.

Lazarowski E. (2003) Molecular and biological properties of P2Y receptors. In: Schwiebert EM (ed) Extracellular nucleotides and nucleosides: release, receptors, and physiological and pathophysiological effects. Academic Press, Amsterdam, Boston, pp 59-96.

Lemoli R. M., Ferrari D., Fogli M., Rossi L., Pizzirani C., Forchap S., Chiozzi P., Vaselli D., Bertolini F., Foutz T., Aluigi M., Baccarani M. and Di Virgilio F. (2004) Extracellular nucleotides are potent stimulators of human hematopoietic stem cells in vitro and in vivo. Blood. 104, 1662-1670. Epub 2004 May 1625.

Lenz G., Gottfried C., Luo Z., Avruch J., Rodnight R., Nie W. J., Kang Y. and Neary J. T. (2000) P(2Y) purinoceptor subtypes recruit different mek activators in astrocytes, in Br J Pharmacol, pp 927-936.

Milosevic J., Schwarz S. C., Krohn K., Poppe M., Storch A. and Schwarz J. (2005) Low atmospheric oxygen avoids maturation, senescence and cell death of murine mesencephalic neural precursors. J Neurochem 92, 718-729.

Mishra S. K., Braun N., Shukla V., Fullgrabe M., Schomerus C., Korf H. W., Gachet C., Ikehara Y., Sevigny J., Robson S. C. and Zimmermann H. (2006) Extracellular nucleotide signaling in adult neural stem cells: synergism with growth factor-mediated cellular proliferation. Development. 133, 675-684.

Neary J. T., Kang Y. and Shi Y. F. (2004) Signaling from nucleotide receptors to protein kinase cascades in astrocytes. Neurochem Res. 29, 2037-2042.

Neary J. T., Kang Y., Willoughby K. A. and Ellis E. F. (2003) Activation of extracellular signal-regulated kinase by stretch-induced injury in astrocytes involves extracellular ATP and P2 purinergic receptors, in J Neurosci, pp 2348-2356.

Neary J. T., Rathbone M. P., Cattabeni F., Abbracchio M. P. and Burnstock G. (1996) Trophic actions of extracellular nucleotides and nucleosides on glial and neuronal cells. Trends Neurosci. 19, 13-18.

Neary J. T., Kang Y., Bu Y., Yu E., Akong K. and Peters C. M. (1999) Mitogenic signaling by ATP/P2Y purinergic receptors in astrocytes: involvement of a calcium-independent protein kinase C, extracellular signal-regulated protein kinase pathway distinct from the phosphatidylinositol-specific phospholipase C/calcium pathway, in J Neurosci, pp 4211-4220.

Nicoletti I., Migliorati G., Pagliacci M. C., Grignani F. and Riccardi C. (1991) A rapid and simple method for measuring thymocyte apoptosis by propidium iodide staining and flow cytometry. J Immunol Methods 139, 271-279.

Palmer R. K., Boyer J. L., Schachter J. B., Nicholas R. A. and Harden T. K. (1998) Agonist action of adenosine triphosphates at the human P2Y1 receptor. Mol Pharmacol. 54, 1118-1123.

Pooler A. M., Guez D. H., Benedictus R. and Wurtman R. J. (2005) Uridine enhances neurite outgrowth in nerve growth factor-differentiated PC12 [corrected]. Neuroscience. 134, 207-214.

Ralevic V. and Burnstock G. (1998) Receptors for purines and pyrimidines. Pharmacol Rev. 50, 413-492.

Rathbone M. P., Middlemiss P. J., Gysbers J. W., Andrew C., Herman M. A., Reed J. K., Ciccarelli R., Di lorio P. and Caciagli F. (1999) Trophic effects of purines in neurons and glial cells. Prog Neurobiol. 59, 663-690.

Ryu J. K., Choi H. B., Hatori K., Heisel R. L., Pelech S. L., McLarnon J. G. and Kim S. U. (2003) Adenosine triphosphate induces proliferation of human neural stem cells: Role of calcium and p70 ribosomal protein S6 kinase. J Neurosci Res. 72, 352-362.

Sak K., Boeynaems J. M. and Everaus H. (2003) Involvement of P2Y receptors in the differentiation of haematopoietic cells. J Leukoc Biol. 73, 442-447.

Scemes E., Duval N. and Meda P. (2003) Reduced expression of P2Y1 receptors in connexin43-null mice alters calcium signaling and migration of neural progenitor cells. J Neurosci. 23, 11444-11452.

Sellers L. A., Simon J., Lundahl T. S., Cousens D. J., Humphrey P. P. and Barnard E. A. (2001) Adenosine nucleotides acting at the human P2Y1 receptor stimulate mitogen-activated protein kinases and induce apoptosis. J Biol Chem. 276, 16379-16390. Epub 12001 Jan 16325.

Shah K., Hsich G. and Breakefield X. O. (2004) Neural precursor cells and their role in neuro-oncology. Dev Neurosci. 26, 118-130.

Storch A., Paul G., Csete M., Boehm B. O., Carvey P. M., Kupsch A. and Schwarz J. (2001) Long-term proliferation and dopaminergic differentiation of human mesencephalic neural precursor cells, in Exp Neurol, pp 317-325.

Wang L., Jacobsen S. E., Bengtsson A. and Erlinge D. (2004) P2 receptor mRNA expression profiles in human lymphocytes, monocytes and CD34+ stem and progenitor cells. BMC Immunol. 5, 16.

Webb T. E., Henderson D. J., Roberts J. A. and Barnard E. A. (1998) Molecular cloning and characterization of the rat P2Y4 receptor. J Neurochem. 71, 1348-1357.

Weissman T. A., Riquelme P. A., Ivic L., Flint A. C. and Kriegstein A. R. (2004) Calcium waves propagate through radial glial cells and modulate proliferation in the developing neocortex. Neuron. 43, 647-661.

Zimmermann H. (2000) Extracellular metabolism of ATP and other nucleotides. Naunyn Schmiedebergs Arch Pharmacol. 362, 299-309.

Zimmermann H. (2006) Nucleotide signaling in nervous system development. Pflugers Arch Eur J Physiol, in press*.*

### Example 2: Dexamethasone promotes proliferation and differentiation of NPCs

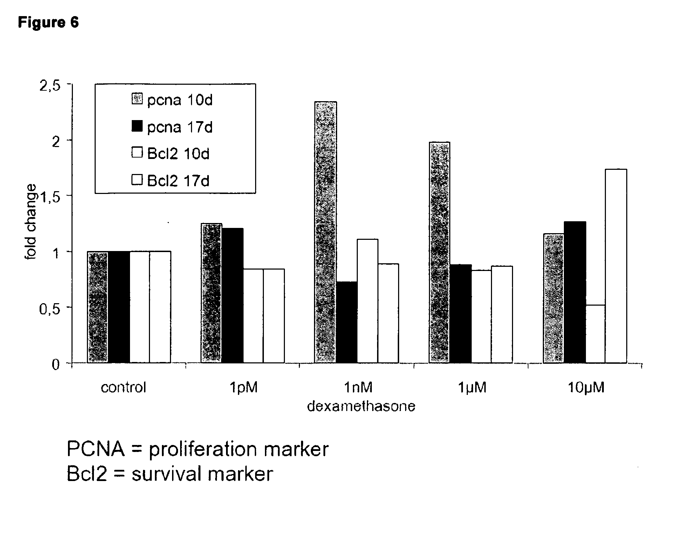

NPCs were incubated with varying concentrations of dexamethasone in culture medium for proliferation. After 10 and 17 days of exposure, expression of the proliferation marker PCNA and of the survival marker Bcl2 was determined. As can be seen from Figure 6, expression of PCNA is strongly increased by dexamethasone.

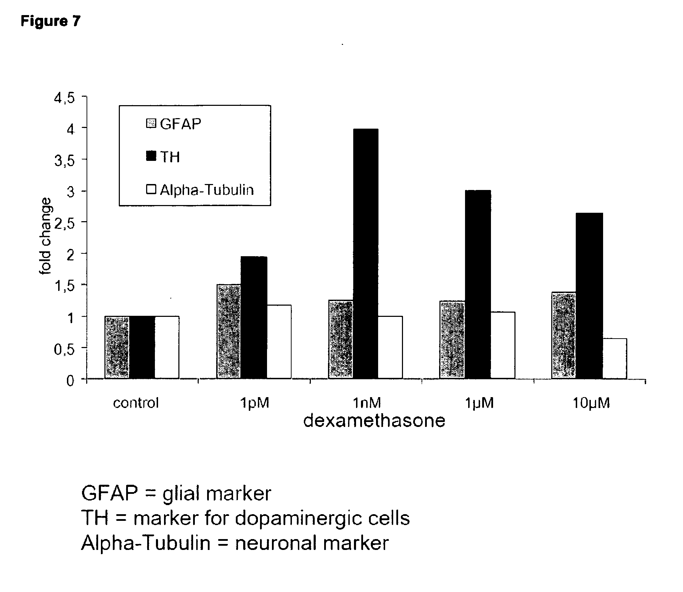

NPCs were incubated with varying concentrations of dexamethasone in culture medium for differentiation. After incubation, expression of GFAP, TH and alpha-tubulin was determined. As can be seen from Figure 7, the expression of the dopaminergic marker TH is strongly enhanced by dexamethasone. At higher concentrations, expression of the glial marker GFAP is reduced.

### Abbreviations

ANOVA, analysis of variance
ADP, adenosine diphosphate
ATP, adenosine triphosphate
DAPI, 6'-diamidino-2-phenylindole
EGF, epidermal growth factor
ERK, extracellular signal-regulated kinase
FGF-2, fibroblast growth factor 2
FCS, fetal calf serum
NGF, nerve growth factors
MAPK, mitogen-activated protein kinase
MEK, MAPK/extracellular protein kinase kinase
hmNPCs, human mesencephalic neural precursor cells
P2Rs, P2 purinoreceptors
PCNA, proliferating cell nuclear antigen
PD, Parkinson's disease
PPADS, Pyridoxal-phosphate-6-azophenyl-2',4'-disulphonic acid
UDP, uridine diphosphate
UTP, uridine triphosphate
TH, tyrosine hydroxylase
Tuj1, Neuronal class III β-tubulin

## Claims

1. A method of culturing neural precursor cells (NPCs), comprising culturing the NPCs in a medium containing at least one uracil nucleotide or dexamethasone.

2. A method according to claim 1 wherein the concentration of the uracil nucleotide in the medium is from 0.01 to 200 µM.

3. A method according to claim1 or 2, wherein the uracil nucleotide is selected from the group consisting of UTP, UDP and combinations thereof.

4. A method according to any one of claims 1 to 3, which comprises differentiating the NPCs into dopaminergic cells in the presence of the uracil nucleotide(s).

5. A method according to any one of claims 1 to 4, which comprises proliferating the NPCs in the presence of the uracil nucleotide(s).

6. A method according to claim 5 wherein the concentration of the uracil nucleotide in the culture medium is from 0.01 to 5 µM.

7. A method according to claim 5 or 6, wherein the culture medium contains one or more growth factors.

8. A method according to claim 7 wherein the growth factor(s) is/are selected from the group consisting of epidermal growth factor (EGF), fibroblast growth factor 2 (FGF-2), and combinations thereof.

9. A method according to any one of the preceding claims wherein the NPCs are human NPCs.

10. A method according to any one of the preceding claims, wherein the hNPCs are derived from the human mid-brain.

11. A cell obtainable by a method according to any one of the preceding claims.

12. A cell according to claim 11, which is a dopaminergic cell.

13. The use of a cell according to claim 11 or 12 for the manufacture of a pharmaceutical composition for treating a neurodegenerative disease and/or neuronal injury.

14. The use of at least one uracil nucleotide for the manufacture of a pharmaceutical composition for treating a neurodegenerative disease and/or neuronal injury.

15. The use according to claim 13 or 14 wherein the neurodegenerative disease is a disease with a predominant loss of dopaminergic neurons.

16. The use according to any one of claims 13 to 15 wherein the neurodegenerative disease is Parkinson's disease.

17. The use of at least one uracil nucleotide and/or of dexamethasone for the proliferation and/or differentiation of NPCs.

18. A population of cells comprising neuronal cells and dopaminergic cells, **characterized in that** the ratio of the number of dopaminergic cells to the number of neuronal cells in the population is at least 0.2.

19. A population of cells comprising neuronal cells and dopaminergic cells, **characterized in that** the ratio of the number of TH-positive cells to the number of MAP-2-positive cells in the population is at least 0.2.
